Europäisches Patentamt

European Patent Office

Office européen des brevets

(11) Veröffentlichungsnummer: **0 220 431**
**A1**

## EUROPÄISCHE PATENTANMELDUNG

(21) Anmeldenummer: **86112190.3**

(22) Anmeldetag: **03.09.86**

(51) Int. Cl.⁴: **A 61 M 1/00**

(30) Priorität: **05.09.85  DE 3531669**

(43) Veröffentlichungstag der Anmeldung: **06.05.87**
**Patentblatt 87/19**

(84) Benannte Vertragsstaaten: **DE FR GB IT**

(71) Anmelder: **Fresenius AG, Gluckensteinweg 5,**
**D-6380 Bad Homburg (DE)**

(72) Erfinder: **Mathieu,Bernd Dr., Galgenbergstrasse 11,**
**D-6683 Spiessen-Elversberg (DE)**

(74) Vertreter: **Görtz, Dr. Fuchs, Dr. Luderschmidt**
**Patentanwälte, Sonnenberger**
**Strasse 100 Postfach 26 26, D-6200 Wiesbaden (DE)**

(54) **Vorrichtung zur Einstellung eines Flusses einer Körperflüssigkeit eines Patienten.**

(57)  Die Erfindung betrifft ein Druckhalteventil (21) für einen portokavalen Shunt (20) mit einem Gehäuse (3), durch das ein elastischer Shuntschlauch (4) geführt ist, und einem Betätigungsglied (24), das von einem unter Vorspannung stehenden Federelement (23) kraftbeaufschlagt ist, wobei das Betätigungsglied (24) zur Querschnittsbeeinflussung des elastischen Shuntschlauches (4) an diesem angreift. Mit dem Druckhalteventil (21) kann auf einfache Weise und zuverlässig das Druckniveau bei einem portokavalen Shunt (20) gehalten werden.

ACTORUM AG

Beschreibung

Vorrichtung zur Einstellung eines Flusses einer
Körperflüssigkeit eines Patienten

Die Erfindung betrifft eine Vorrichtung zur Einstellung eines Flusses einer Körperflüssigkeit eines Patienten durch einen intrakorporal plazierten Shuntschlauch zwischen einem ersten und einem zweiten Körpergefäß mit einem Druckhalteventil, in dem der Shuntschlauch angeordnet ist und das Mittel zur Veränderung des Schlauchquerschnitts aufweist.

Patienten mit Leberzirrhose leiden im fortgeschrittenen Stadium ihrer Krankheit an einer Verengung der venösen Kapillaren, die von der Pfortader ausgehend, das Lebergewebe durchziehen. Das mit Fetten und anderen abzubauenden Stoffen beladene Blut aus dem Darmbereich kann so die Leber nicht mehr ungehindert durchfließen und staut sich in der Pfortader auf. Der normalerweise dort herrschende Druck wird dadurch bei weitem überschritten und pflanzt sich rückwärts in das venöse System fort. Durch diesen Überdruck kommt es in sehr vielen Fällen zu Varizen (Krampfadern) an den verschiedensten Stellen. Während diese z. B. auf der Bauchdecke relativ ungefährlich sind, können sie in der Speiseröhre oder im Magen lebensbedrohend sein. Bei einer Blutung durch Platzen des Gefäßes kann es zu einer Verblutung kommen, bevor der Patient in ärztliche Behandlung gelangt. Diejenigen, die die erste Blutung überleben, werden nach zwei Methoden behandelt.

Als erstes werden die akuten Varizen verödet und somit die Blutung gestoppt. Danach gibt es zwei Möglichkeiten: Die Varizenverödung wird regelmäßig solange weitergeführt, bis sie an Stellen auftauchen, an denen sie nicht mehr behandelbar sind (Magenfundus), oder ein s. g. portaler Shunt wird operativ ausgeführt. Diese Operation besteht aus dem Verbinden eines Gefäßes dieser druckbelasteten Region mit einem anderen, das das Blut zum Herzen führt.

Derartige Shunt-Operationen zur Druckableitung von der Pfortader werden nach verschiedenen Techniken ausgeführt. Alle diese Techniken haben spezifische Vor- und Nachteile. Die Verbindung der Pfortader mit der Milzvene oder anderen kleinen venösen Gefäßen neigt zum Verschluß, die Verbindung zur Venacava bleibt zwar offen, senkt aber das Druckniveau vor der Leber so stark, daß die Leber nicht ausreichend durchflossen wird und das Blut damit unentgiftet direkt zum Herzen gelangt. Dies hat zur Folge, daß zwar die Varizen zurückgehen und die Gefahr eines Verblutens gebannt ist, daß aber der Patient im Laufe der Zeit durch eine Enzephalopatie hirnorganisch geschädigt wird.

Eine Vorrichtung der eingangs erwähnten Art wird bei der Behandlung des Hydrozephalus eingesetzt. Dabei wird der unter Druck stehende Schädelinnenraum über einen Katheterschlauch mit einer Körpervene verbunden, wobei in den Katheterschlauch ein Rückschlagventil eingeschaltet ist, das den Abfluß der zum Überdruck führenden Flüssigkeit in die Vene zuläßt (Sog. Drainage nach Spitz-Holter). Diese Ventrikeldrainage gestattet somit ebenfalls einen Druckausgleich zwischen zwei Körpergefäßen wie er mit dem vorstehend beschriebenen portokavalen Shunt erreicht wird.

Aus der deutschen Offenlegungsschrift 21 17 131 ist eine Vorrichtung zum Steuern einer Gefäßverbindung beschrieben, die aus einem Shunt und einer diesen Shunt umgebenden Ventileinrichtung besteht. Diese Ventileinrichtung kann durch eine außerhalb des Körpers des Patienten befindliche Steuervorrichtung verengt oder erweitert werden und verbleibt fest bis zur nächsten Einstellung in der eingestellten Position. Infolgedessen kann sich diese Ventileinrichtung nicht selbsttätig an sich verändernde Strömungsverhältnisse anpassen. Darüberhinaus besteht die Gefahr, daß bei einer Schließung des Ventils und somit der Aufhebung des Blutdurchflusses es zu einem Gerinnen des Blutes kommt, das sich im Absperrbereich befindet. Die sich hierdurch bildenden Gerinsel können beim anschließenden Öffnen des Ventils entweder den gesamten Shuntschlauch verstopfen, somit also die Vorrichtung unwirksam machen oder aber eine ernsthafte Gefahr für die Gesundheit des Patienten darstellen, sofern sie durch den wiederhergestellten Blutfluß fortgeschwemmt werden.

Aus dem schweizerischen Patent 463 015 ist wiederum eine intrakorporale Verschlußeinrichtung für rohrförmige Gefäße bekannt, die ebenfalls ein Körpergefäß völlig verschließen kann. Insofern treten auch hier die vorstehend erwähnten Gerinnungsprobleme auf.

Der Erfindung liegt daher die Aufgabe zugrunde, die Vorrichtung der eingangs erwähnten Art so fortzubilden, daß sie den Fluß der Körperflüssigkeit selbsttätig regelt.

Eine weitere Aufgabe der Erfindung besteht darin, die bekannte Vorrichtung so fortzubilden, daß bei ihr die vorstehend erwähnten Gerinnungsprobleme vermieden werden.

Diese Aufgaben werden dadurch gelöst, daß das Druckhalteventil ein Gehäuse mit einer Öffnung , in dem die Mittel zur Veränderung des Schlauchquerschnitts angeordnet sind, und mit einer gegenüber der Öffnung engeren Ausnehmung aufweist, in der der Schlauch angeordnet ist, wobei die Tiefe der Ausnehmung etwas größer ist als die doppelte Wandstärke des Schlauchs. und daß die Mittel zur Veränderung des Schlauchquerschnitts gegen die sich seitlich von der Ausnehmung anschließenden Anschlagbereiche elastisch abgestützt sind.

4

Mit der erfindungsgemäßen Lösung wird ein Druckhalteventil für einen portocavalen Shunt geschaffen, das dafür sorgt, daß das Druckniveau nicht unzulässig abgesenkt wird, so daß bei einer Einpflanzung des Druckhalteventils zwischen Pfortader und Vena cava gewährleistet ist, daß in der Pfortader ein Mindestdruck von beispielsweise 20 mm Hg gehalten wird. Dadurch ist für eine ausreichende Durchblutung der Leber gesorgt und die Varizenblutung trotzdem verhindert. Erst bei Überschreiten eines bestimmten Druckniveaus von beispielsweise 20 mm Hg wird die überschüssige Blutmenge zur Hohlvene abgeleitet.

Gemäß einer bevorzugten Ausführungsform der Erfindung ist vorgesehen, daß das Gehäuse eine ovale bzw. elliptische Form aufweist. Der größte Durchmesser liegt dabei zweckmäßig im Bereich von 2 - 4 cm, während die Dicke etwa 5 - 6 mm beträgt.

Um eine einfache Befestigung des Shuntschlauches mit dem Druckhalteventil an den entsprechenden Körpergefäßen zu ermöglichen, weist der Shuntschlauch an seinen aus dem Gehäuse heraustretenden Enden trichterförmige Befestigungsabschnitte auf, die insbesondere mit einem Belag versehen sind, der beispielsweise aus Dacronvelour besteht.

Gemäß einer weiteren bevorzugten Ausführungsform der Erfindung weist der Shuntschlauch im Bereich des Betätigungsgliedes einen flachen, insbesondere rechteckförmigen oder ovalen Querschnitt auf. Dieser Querschnitt kann zur Bildung eines Anschlages für das Betätigungsglied teilweise in einer Vertiefung des Gehäuses angeordnet sein.

Das Betätigungsglied selbst ist zweckmäßig als plattenförmiges Element ausgebildet, welches über dem zu verengenden Querschnitt des Shuntschlauches angeordnet ist.

Zwischen diesem Betätigungsglied und dem Gehäuse ist mit Vorteil die das Betätigungsglied kraftbeaufschlagende Feder angeordnet, die zweckmäßig als gewellte Blattfeder ausgebildet ist, die insbesondere mit ihren Endbereichen am Betätigungsglied abgestützt ist. Um die Wirksamkeit der Feder nicht zu beeinträchtigen, sind die Federenden, mit denen die Feder am Betätigungsglied abgestützt ist, umgebogen. Der zu verengende Querschnitt, das Betätigungsglied und die Feder sind zweckmäßig in einer Ausnehmung des Ventilgehäuses angeordnet, die insbesondere einen im wesentlichen rechteckigen Querschnitt aufweist und dem Betätigungsglied und der Feder größenmäßig insoweit angepaßt ist, daß zusätzliche Halterungen für das Betätigungselement und die Feder entfallen können.

Als Werkstoffe für die einzelnen Bauteile kommen insbesondere in Frage: für das Gehäuse Kunststoff, für den Shuntschlauch Silikongummi, für die Feder Edelstahl, für das Betätigungsglied Teflon (PTFE).

Weitere Vorteile und Merkmale der Erfindung ergeben sich aus der nachfolgenden Beschreibung eines Ausführungsbeispiels in Verbindung mit der Zeichnung.

Es zeigen:

Fig. 1   eine Draufsicht auf einen portokavalen Shunt mit Druckhalteventil;

Fig. 2   eine Vorderansicht des in Fig. 1 dargestellten Shunts mit Druckhalteventil;

Fig. 3  einen Schnitt durch den in den Figuren
        1 und 2 dargestellten Shunt;

Fig. 4  einen Längsschnitt ohne Befestigungsabschnitte durch den in den Figuren 1 bis 3
        dargestellten Shunt;

Fig. 5  einen Schnitt, entsprechend dem Schnitt von

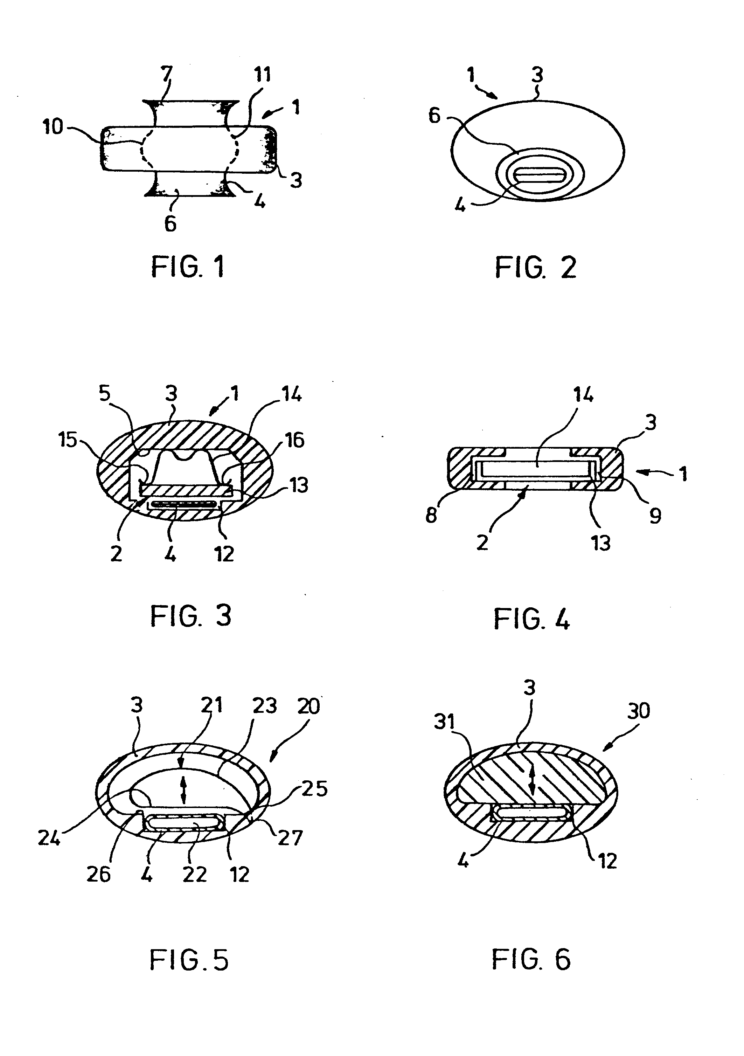

        Fig. 3 durch eine zweite Ausführungsform und

Fig. 6  einen weiteren Schnitt entsprechend dem
        Schnitt von Fig. 3 durch eine dritte Ausführungsform.

Das in  Fig. 1-4   dargestellte Ausführungsbeispiel zeigt einen Shunt  1, mit einem Druckhalteventil 2. Das Druckhalteventil 2 weist ein Gehäuse 3
auf, durch das ein elastischer  Shuntschlauch
4 geführt ist. Das Gehäuse 3 ist von ovaler bzw.
ellipsenförmiger Gestalt, wobei sein größerer
Durchmesser größenordnungsmäßig 3 bis 4 cm und
sein kleinerer Durchmesser größenordnungsmäßig
2 bis 3 cm beträgt. Die Dicke des Gehäuses liegt
im Bereich weniger mm, insbesondere etwa bei
6 bis 10 mm. Im Gehäuse 3 ist eine durchgehende
Öffnung 5, die im mittleren Bereich einen im
wesentlichen rechteckförmigen Querschnitt aufweist.
Durch diese Öffnung ist der Shuntschlauch 4 durchgeführt. Der Shuntschlauch 4 weist an seinen aus
dem Gehäuse 3 herausragenden Enden 6, 7 trichterförmige Befestigungsabschnitte 6, 7 zur Verbindung
mit den entsprechenden Körpergefäßen auf. Im
Bereich der rechteckigen Querschnitte des Gehäuses
3 sind seitliche Ausnehmungen 8,9 ausgebildet. Der
Shuntschlauch 4 weist in diesem Bereich Ausformungen 10, 11 auf, die in die Ausnehmungen 8, 9
hineinragen, so daß der Schlauch auf diese Weise
gegenüber dem Gehäuse 3 fixiert ist.

7

Wie insbesondere aus Figur 3 deutlich ersichtlich ist, ist im Gehäuse 3 eine Ausnehmung 12 vorgesehen, in der der in diesem Bereich flache Shuntschlauch 4 mit einem Teil seines Querschnittes angeordnet ist. Über diesem Teil des Shuntschlauches 4 ist ein als Andruckplatte 13 ausgebildetes Betätigungsglied angeordnet, dessen Enden in die Gehäuseausnehmungen 8, 9 ragen, so daß die Andruckplatte im Gehäuse in Richtung der Shuntachse und in Richtung ihrer Längsachse mit geringem Spiel, jedoch im wesentlichen unverschiebbar, geführt ist. Gegen die Andruckplatte 13 wirkt eine Feder 14, die als gewellte Blattfeder ausgeführt ist. Die umgebogenen Enden 15, 16 der Blattfeder 14 stützen sich dabei an der Andruckplatte 13 ab, wohingegen der mittlere Bereich der Feder am oberen Teil des Gehäuses 3 anliegt. Durch die Feder wird der Shuntschlauch 4 zusammengedrückt. Die Feder hat dabei eine Vorspannung, die erst bei einem bestimmten Druck von beispielsweise 20 mm $_{Hg}$ überwunden wird. Von da an ist ihr Kraftverlauf sehr flach. Dies führt dazu, daß durch relativ geringe Druckerhöhungen eine große Querschnittszunahme erreicht wird.

Durch die Anordnung des Shuntschlauches 4 in der Gehäuseausnehmung wird für die Andruckplatte 13 durch die seitlichen Begrenzungen der Ausnehmung 12 ein Anschlag gebildet. Die vorgespannte Feder wird dadurch daran gehindert, den Shuntschlauch vollständig zu quetschen. Daher ist stets ein bestimmter Minimalfluß auch bei "geschlossenem" Ventil gewährleistet. Durch die Breite der Feder und damit die Länge des offenstehenden Kanals und durch die Höhe des Spalts kann die Blutmenge festgelegt werden, die immer durch das Ventil fließt. Dieser konstante Fluß ist gegebenenfalls notwendig, um eine Gerinnung des Blutes und ein Verkleben des Ventils zu vermeiden.

In Fig. 5 ist eine weitere Ausführungsform eines Shunt 20 gezeigt, bei dem die gleichen Bezugszeichen für die gleichen Teile gem. Fig. 1 - 4 verwendet werden. Demzufolge ist ein biegeschlaffer Shuntschlauch 4 in dem Gehäuse 3 vorgesehen. Dieses Gehäuse 3 weist wiederum eine Ausnehmung 12 auf, in der der Shuntschlauch 4 angeordnet ist. Die Tiefe der Ausnehmung 12 ist so bemessen, daß sie den Shuntschlauch 4 aufnehmen kann, ohne daß dieser durch die Feder 21 völlig zusammengedrückt wird. Der erfindungsgemäß eingesetzte Shuntschlauch weist üblicherweise - entgegen den elastisch sich rückstellenden Schläuchen - praktisch keine Rückstellkraft auf, d. h. er ist aus einem schlaffen Material gefertigt. Insofern kann der Shuntschlauch 4 im wesentlichen ohne Krafteinwirkung völlig zusammengedrückt werden mit der Folge, daß die Berücksichtigung elastischer Rückstellkräfte bei dem erfindungsgemäß eingesetzten Shuntschlauch 4 im wesentlichen zu vernachlässigen sind.

Insofern ist also erfindungsgemäß die Tiefe der Ausnehmung 12 etwas größer als die doppelte Wandstärke des Shuntschlauchs 4, so daß der Shuntschlauch 4 bebreits durch einen Flüssigkeitsstrom mit einem sehr geringen Druckgefälle unter Bildung eines Strömungsschlitzes 22 - wie dies in Fig. 5 gezeigt ist - geöffnet werden kann. Hierdurch wird der vorstehend beschriebene Minimalfluß gewährleistet. Erst ab einem bestimmten Strömungsdruck, der zur Erweiterung des Shuntschlauchs 4 führt, kommt dieser mit der Feder 21 in Berührung, die in der in Fig. 5 gezeigten Ausführungsform als halbkreisförmige Blattfeder 23 ausgebildet ist, die einstückig in die Andruckplatte 24 übergeht. Das andere Ende 25 der Blattfeder 23 ist im Gehäuse 3 verankert und dort mit einer bestimmten Vorspannung festgelegt. Dies führt dazu, daß die Andruckplatte 24 auf den seitlich von der Ausnehmung 12 gebildeten Anschlagbereichen 26, 27 aufliegt und erst

0220431

ab einem bestimmten Druck, beispielsweise 15 - 20 mm Hg angehoben werden kann.

Erfindungsgemäß gewährleistet das Druckhalteventil 21 stromauf, d. h. in Zuflußrichtung im Shuntschlauch 4 einen Strömungsdruck von 15 - 40, vorzugsweise 20 - 25 mm Hg in der Körperflüssigkeit, üblicherweise Blut.

Gem. einer weiteren, nicht gezeigten Ausführungsform können anstelle einer halbkreisförmig ausgebildeten Blattfeder 23 auch zwei halbkreisförmig ausgebildete Blattfedern 23 unter Bildung eines vollen Kreises eingesetzt werden, wobei die Andruckplatten 24 oberhalb und unterhalb des Shuntschlauchs 4 angeordnet sind.

In Fig. 6 ist eine weitere Ausführungsform eines Shunts 30 gezeigt, dessen Gehäuse 3 im wesentlichen dem Gehäuse gem. Fig. 3 und 5 entspricht, also ebenfalls eine Ausnehmung 12 innerhalb einer im wesentlichen elliptisch ausgebildeten Gehäuseform aufweist. In dieser Ausnehmung 12, die jedoch nicht notwendigerweise vorgesehen sein muß, ist wiederum der Shuntschlauch 4 angeordnet. Der restliche im Gehäuse verbliebene Zwischenraum ist mit einer elastischen Masse 31, beispielsweise einer geschäumten Kunststoffmasse (Moosgummi) oder einem gummielastischen Vollmaterial (Silikonkautschuk) gefüllt, die in Richtung des gezeigten Doppelpfeils sich elastisch bewegen kann. Dabei wird die Federeigenschaft dieser elastischen Masse 31 entsprechend dem vorstehend zu erzielenden Strömungsdruck von 15 - 40, vorzugsweise 20 - 25 mm Hg gewählt.

Auch gem. dieser Ausführungsform ist gewährleistet, daß die elatische Masse 31 als Federschicht erst dann in Aktion tritt, wenn eine bestimmte Lumenbreite im Shuntschlauch 4 überschritten wird.

Bezüglich der zu verwenden Materialien ist anzumerken, daß diese einerseits funktionsgerecht ausgewählt werden müssen, jedoch gewährleistet sein muß , daß ausschließlich pharmazeutisch unbedenkliche Materialien verwendet werden.

Die gesamte Oberfläche des Implanats muß gewebeverträglich sein und darf keine Stoffe an das umgebende Gewebe abgeben. Für das Gehäuse 3 kommt insbesondere Kunststoff in Frage, der Shuntschlauch 4 kann zweckmäßig aus Silikongummi bestehen. Für die Andruckplatte 13 erscheint Teflon zweckmäßig, während die Feder aus Edelstahl besteht.

Gem. der in Fig. 5 gezeigten Ausführungsform besteht die Feder 23 und die Andruckplatte 24 aus dem gleichen Material, also Edelstahl oder Edelmetall (Gold).

Im übrigen ist das gesamte Druckhalteventil innerhalb des Gehäuses verkapselt angeordnet und von diesem umgeben, wobei das Gehäuse 3 - wie vorstehend beschrieben ist - aus einem gewebeverträglichen Material (Dacronvelour) umfaßt ist. Vorteilhafterweise ist der Shuntschlauch in seinem jeweiligen Austrittsbereich aus dem Gehäuse 3 mit dem Gehäuse 3 vergossen, so daß keine Gewebeflüssigkeit oder Gewebe selbst in das Gehäuse 3 eindringen und die Funktion des Druckhalteventils beeinträchtigen kann.

Das Öffnen des Ventils erfolgt vorteilhaft bei etwa 20 mm Hg. Bei einem Druck unterhalb dieses Niveaus erfolgt ein Blutfluß von etwa 50 ml/min durch das Ventil. Der Druck wird zweckmäßig auf 30 mm Hg begrenzt. Ein Vordruck von über 50 mm Hg wird in jedem Falle abgeleitet.

- 11 -

Wie bereits vorstehend bei der Ausführungsform gemäß Fig. 5 beschrieben, ist die Tiefe der Ausnehmung 12 etwas größer als die doppelte Wandstärke des Shuntschlauchs 4, der aufgrund seiner biegeschlaffen Eigenschaften im wesentlichen ohne Krafteinwirkung völlig zusammengedrückt werden kann und sich schon bei geringen Drücken erweitert. Erfindungsgemäß ist die Tiefe der Ausnehmung so dimensioniert, daß bei Drücken bis zu 15 - 20 mm/Hg der freie Querschnitt des Shuntschlauchs in der Ausnehmung ausreicht, um den Blutfluß aufzunehmen, ohne daß das Ventil betätigt wird. Bei entsprechender Dimensionierung des Shuntschlauchquerschnitts in dieser Ausnehmung kann dann ein Blutfluß bis zu etwa 50 ml/min ohne Betätigung des Ventil zugelassen werden.

Dieser freie Schlauchquerschnitt hängt aber im wesentlichen von der Breite und der Tiefe der Ausnehmung ab, wobei unterstellt wird, daß der Shuntschlauch im wesentlichen den Abmessungen der Ausnehmung angepaßt ist.

Mit dem Druckhalteventil 1 kann entweder eine side-byside-Anastomose oder aber auch eine end-to-side-Anastomose durchgeführt werden.

Desweiteren kann das Druckhalteventil 1 zur direkten Verbindung der vena cava mit der von der Leber abgetrennten Pfortader eingesetzt werden, wobei der arterielle Kreislauf der Leber aufrechterhalten bleibt.

0220431

Patentansprüche

1. Vorrichtung zur Einstellung eines Flusses einer Körperflüssigkeit eines Patienten durch einen intrakorporal plazierten Shuntschlauch zwischen einem ersten und einem zweiten Körpergefäß mit einem Druckhalteventil, in dem der Shuntschlauch angeordnet ist und das Mittel zur Veränderung des Schlauchquerschnitts aufweist, dadurch gekennzeichnet, daß das Druckhalteventil (2, 21, 31) ein Gehäuse (3) mit einer Öffnung (5), in dem die Mittel zur Veränderung des Schlauchquerschnitts angeordnet sind, und mit einer gegenüber der Öffnung (5) engeren Ausnehmung (12) aufweist, in der der Schlauch (4) angeordnet ist, wobei die Tiefe der Ausnehmung (12) etwas größer ist als die doppelte Wandstärke des Schlauchs (4) und daß die Mittel zur Veränderung des Schlauchquerschnitts gegen die sich seitlich von der Ausnehmung (12) anschließenden Anschlagbereiche (26, 27) elastisch abgestützt sind.

2. Vorrichtung nach Anspruch 1, dadurch gekennzeichnet, daß die Mittel zur Veränderung des Schlauchquerschnitts als Federelement (14, 23) ausgebildet sind und eine derartige Federcharakteristik aufweisen, daß der Eingangsdruck der Körperflüssigkeit in einem Bereich von 15 - 40 mm/Hg, vorzugsweise 20 - 25 mm Hg gehalten wird.

0220431

3. Vorrichtung nach Anspruch 2, d a d u r c h g e k e n n z e i c h n e t, daß das Federlement (14, 23) zwischen einer Andruckplatte (13, 24) und der Gehäuseinnenwand in der Öffnung (5) des Gehäuses (3) gespannt vorgesehen ist.

4. Vorrichtung nach Anspruch 2 oder 3, d a d u r c h g e k e n n z e i c h n e t, daß die Feder (14, 23) als gewellte oder halbkreisförmig gebogene Blattfeder ausgebildet ist.

5. Vorrichtung nach Anspruch 2 oder 3, d a d u r c h g e k e n n z e i c h n e t, daß das Federelement eine elastische Masse (31) ist, die in der Öffnung (5) des Gehäuses (3) mit Ausnahme der Ausnehmung (12) vorgesehen ist.

6. Vorrichtung nach Anspruch 5, d a d u r c h g e k e n n z e i c h n e t, daß die elastische Masse (31) eine gegebenenfalls geschäumte, elastisch rückstellende Kunststoffmasse ist.

7. Vorrichtung nach einem der Ansprüche 1 - 6, d a d u r c h g e k e n n z e i c h n e t, daß das Gehäuse (3) eine Ausnehmung (12) zur Aufnahme des Shuntschlauchs (4) aufweist, deren Tiefe etwas größer ist als die doppelte Wandstärke des Shuntschlauchs.

8. Vorrichtung nach Anspruch 7, d a d u r c h g e - k e n n z e i c h n e t, daß die Andruckplatte (13, 24) auf den von der Ausnehmung (12) gebildeten Anschlagbereichen (26, 27) aufliegt.

9. Vorrichtung nach einem der Ansprüche 1 - 8, d a d u r c h g e k e n n z e i c h n e t, daß der Shuntschlauch (4) durch die im Gehäuse (3) vorgesehene Öffnung (5) geführt ist und in dem Gehäuse (3) verkapselt eingegossen ist und jeweils an seinen Enden trichterförmige Befestigungsabschnitte (6,7) aufweist.

0220431

FIG. 1

FIG. 2

FIG. 3

FIG. 4

FIG. 5

FIG. 6

0220431

Nummer der Anmeldung

**Europäisches Patentamt**

**EUROPÄISCHER RECHERCHENBERICHT**

## EINSCHLÄGIGE DOKUMENTE

EP 86112190.3

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | CH - A - 463 015 (P. PLISHNER)<br>* Gesamt; insbesondere Fig. 3,4; Patentansprüche 1,5 * | 1,7 | A 61 M 1/00 |
| A | | 2 | |
| Y | FR - A - 2 062 329 (ILLINOIS TOOL)<br>* Gesamt; insbesondere Fig. 3,9, 15 * | 1,7 | |
| A | US - A - 3 769 982 (R. SCHULTE)<br>* Gesamt; insbesondere Fig. 19-22; Spalte 11, Zeilen 54-63 * | 1 | |
| A | US - A - 4 406 440 (L. KULLE et al.)<br>* Gesamt; insbesondere Fig. 2 * | 1 | RECHERCHIERTE SACHGEBIETE (Int. Cl.4) |
| D,A | DE - A - 2 117 131 (AGENCE NAT.)<br>* Gesamt *<br>---- | 1 | A 61 F 2/00<br>A 61 B 17/00<br>A 61 M 1/00<br>A 61 M 5/00 |

Der vorliegende Recherchenbericht wurde für alle Patentansprüche erstellt.

| Recherchenort | Abschlußdatum der Recherche | Prüfer |
|---|---|---|
| WIEN | 21-01-1987 | LUDWIG |